# EUROPÄISCHE PATENTSCHRIFT

(11) **EP 0 741 574 B1**
(45) Veröffentlichungstag und Bekanntmachung des Hinweises auf die Patenterteilung: **05.06.2002**
(21) Anmeldenummer: 95904434.8
(22) Anmeldetag: 08.12.1994
(51) Int. Cl.: A61K 47/48, A61K 31/196, A61K 31/192, A61K 31/401

(54) **ORALE ARZNEIFORM ENTHALTEND COLESTIPOL ALS TRÄGER MIT SAUREN WIRKSTOFFEN UND VERFAHREN ZU IHRER HERSTELLUNG**
ORAL DOSAGE FORM CONTAINING COLESTIPOL AS A CARRIER AND ACIDIC ACTIVE SUBSTANCES AND A PROCESS FOR PRODUCING THE SAME
FORME GALENIQUE POUR ADMINISTRATION PAR VOIE ORALE CONTENANT DU COLESTIPOL COMME EXCIPIENT ET DES PRINCIPES ACTIFS ACIDES, ET SON PROCEDE DE PREPARATION

(30) Priorität: 27.01.1994 DE 4402379
(43) Veröffentlichungstag der Anmeldung: 13.11.1996
(73) Patentinhaber: LTS Lohmann Therapie-Systeme AG, 56626 Andernach (DE)
(72) Erfinder: MÜLLER, Walter, DE-56564 Neuwied (DE); CREMER, Karsten, DE-53119 Bonn (DE)
(74) Vertreter: Flaccus, Rolf-Dieter, Dr.
(86) Internationale Anmeldenummer: EP9404082
(87) Internationale Veröffentlichungsnummer: WO9520394

(56) Entgegenhaltungen:
- EP-A- 0 010 299
- EP-A- 0 087 864
- EP-A- 0 409 432
- US-A- 4 221 778
- J. PHARM. PHARMACOL., Bd. 46, Januar 1994 Seiten 73-5, EL-SAYED ET AL 'The Effect of Colestipol and Cholestyramine on the Systemic Clearance of Intravenous Ibuprofen in Rabbits'
- INT. J. CLIN. PHARMACOL. AND THER., Bd. 32, no.8, August 1994 Seiten 441--5, AL-BALLA ET AL 'The Effects of Chlestyramine and Colestipol on the Absorption of Diclofenac in Man'
- REYES A. J. 'Diuretics: Clinical Pharmacology and Uses in Cardiovascular Medicine, Nephrology and Hepatology' , GUSTAV FISCHER VERLAG , 1992 Bd. 9, Pharmacology of Furosemide in Healthy Man, p.147-171 siehe Seite 153, Zeile LETZTE - Seite 154, Zeile 15

## Beschreibung

Die vorliegende Erfindung betrifft eine orale Arzneiform mit einem Träger für Wirkstoff, bei dem der Wirkstoff über mindestens eine freie Carboxylgruppe verfügt, die ionisch an ein Polymer mit tertiären Aminogruppen gebunden ist, sowie ein Verfahren zu ihrer Herstellung.

Die Bindung von ionisierbaren Substanzen an Ionenaustauscherharze ist nicht neu und wird in den Standardwerken der pharmazeutischen Technologie als eine Möglichkeit erwähnt, orale Arzneiformen zu erhalten, die über eine verlängerte Freisetzung verfügen. In dem U.S. Patent No. 2,990,332 wird ein solches orales System mit verlängerter Freisetzungszeit beschrieben. Bei einem solch einfachen Ionentauscherharz-Wirkstoff-Komplex erfolgt die Wirkstoffabgabe allerdings nur über einen relativ kurzen Zeitraum und nicht über einen für ein Retardpräparat notwendigen Mindestzeitraum von etwa 4 Stunden.

Verbesserungen werden in den U.S. Patenten No. 3,138,525; 3,499,960 und 3,594,470, dem belgischen Patent Nr. 729,827 und dem deutschen Patent Nr. 2,246,037 beschrieben. Gemäß diesen Patenten können die Ionentauscherteilchen zur Verlängerung der Abgabezeit mit einem zusätzlichen Überzug zur Steuerung der Wirkstoffabgabe versehen werden. Im U.S. Patent Nr. 4,221,778 wird vorgeschlagen, die Ionentauscherteilchen nach der Beladung mit Wirkstoff und vor dem Beschichten mit der Steuermembran mit einem Mittel zu imprägnieren, das bei Kontakt der Ionentauscherteilchen mit Wasser bzw. Magen-Darmsaft ein übermäßiges Quellen dieser Teilchen und eine damit verbundene Zerstörung der Steuermembran verhindert.

Eine andere Anwendung von Ionenaustauschern ist die Bindung von pharmazeutischen Wirkstoffen, um deren unangenehmen Geschmack (DE-OS 3028082) bzw. deren unangenehmen Eigengeruch (CH-PS-3 83 552) günstig zu beeinflussen.

Unbeladene basische Ionenaustauscher finden als Medikamente zur Senkung des Blutcholesterinspiegels schon seit langer Zeit Verwendung. Ihre Wirkung beruht darauf, daß sie im Darmtrakt Gallensäuren binden und zur Ausscheidung bringen. Die dadurch verhinderte Resorption führt dazu, daß der sogenannte enterohepatische Kreislauf unterbrochen wird. Der Organismus reagiert darauf mit einer verstärkten Umwandlung von Cholesterin in Gallensäuren mit der direkten Folge, daß der Blutcholesterinspiegel sinkt.

E1-Sayed et al, J. Pharm. Pharmacol. 46, 73-75 (1994) beschreiben den Effekt der beiden Ionenaustauscher Colestipol und Cholestyramin auf die pharmakokinetischen Parameter von Ibuprofen. Um unerwünschte Interaktionen mit gleichzeitig verabreichten sauren Wirkstoffen wie Ibuprofen, näher zu untersuchen, wurden Colestipol oder Cholestyramin eine halbe Stunde vor intravenöser Ibuprofen-Gabe oral verabreicht. Dabei zeigte sich, daß Cholestyramin, nicht jedoch Colestipol, die Eliminierung von Ibuprofen beschleunigte.

Einen vergleichbaren Effekt beschreiben Al-Balla et al, Int. J. Clin. Pharmacol. and Ther. 32, 441-445 (1994) bei einer unmittelbar auf die orale Gabe von Colestipol oder cholestyramin folgenden Diclofenac-Einnahme. Ebenso zeigen du Souich et al, in: Diuretics: Clinical Pharmacology and uses in cardiovascular medicine, nephrology and hepatology, p 147-171, Gustav Fischer Verlag, 1992, daß Colestipol oder Cholestyramin bei gleichzeitiger Gabe die Bioverfügbarkeit des Diuretikums Furosemid verringert.

In den europäischen Patentanmeldungen EP 0 .010 299 A 1, EP 0 087 864 A 3 und EP 0 508 665 A2 wird die gleichzeitige Verabreichung von Colestipol oder anderen "bile salt bindern" mit Inhibitoren der Cholesterolbiosynthese, mit essentiellen Fettsäuren oder mit Inhibitoren des Angiotensinkonvertierenden Enzyms vorgeschlagen. Durch die synergistischen Wirkungen dieser unterschiedlichen WirkstoffKombinationen soll der Cholesterolspiegel im Blutserum herabgesetzt werden.

Colestipol selber ist als wirksamer Bestandteil einer neuen Arzneiform in EP 0 409 432 A 3, offenbart, die zum Ziel hat, eine Geschmacksverbesserung des Colestipols zu erzielen.

Der Erfindung liegt die Aufgabe zugrunde, eine orale Arzneiform mit sauren Wirkstoffen und ein Verfahren zu ihrer Herstellung anzugeben, in der bei hoher Austauschkapazität eines Ionenaustauschträgers flüssige sowie tiefschmelzende Wirkstoffe ohne zusätzliche Lösemittel in rieselfähiges, temperaturempfindliches Granulat überführt werden können, mit welchen sich bei im sauren Magensaft schwer löslichen Wirkstoffen höhere Konzentrationen an gelöstem Wirkstoff erzielen lassen, und wobei durch gezielte Maßnahmen eine rasche oder retardierte Freisetzung des Wirkstoffes aus der Arzneiform erreichbar ist.

Es wurde nun überraschenderweise gefunden, daß einer dieser in der Medizin schon lange gebräuchlichen Ionenaustauscher, Colestipol bzw. Colestipolyhydrochlorid, sich ausgezeichnet als Träger für saure Wirkstoffe eignet und dabei für die Arzneiform folgende Vorteile in sich vereint:
a. hohe Austauschkapazität
b. flüssige Wirkstoffe können ohne zusätzliche Hilfs- und Lösemittel in ein gut rieselfähiges Granulat überführt werden
c. tiefschmelzende Wirkstoffe können aus der Schmelze ohne die Verwendung von zusätzlichen Lösemitteln in ein praktisch temperaturunempfindliches Granulat überführt werden
d. gutes Fließverhalten des Granulats im unbeladenen und beladenen Zustand
e. die Freisetzung aus dem Wirkstoff/Träger-Komplex erfolgt sehr rasch und erfüllt bzw. übertrifft die an eine schnell zerfallende Arzneiform gestellten Anforderungen
f. es stellen sich u.U. bei im sauren Magensaft schlecht löslichen Wirkstoffen höhere Konzentrationen an gelöstem Wirkstoff ein
g. durch zusätzliche Maßnahmen ist eine verlängerte Freisetzung zu erreichen
h. erwiesene toxikologische Unbedenklichkeit durch langjährige Erfahrungen bei hoher Dosierung zur Behandlung von krankhaft hohen Cholesterinspiegeln

Colestipolhydrochlorid ist chemisch gesehen ein quervernetztes Polymer aus Diethylentriamin und 1-Chlor-2,3-epoxypropan, bei dem jedes 5. Stickstoffatom synthesebedingt protoniert vorliegt.

Falls erforderlich kann Colestipolhydrochlorid aber auch ohne Probleme für den Gebrauch im Sinne dieser Erfindung durch eine Behandlung mit wässrigen Basen vollständig in die freie Base überführt werden.

Die therapeutische Dosis Colestipolhydrochlorid zur Senkung des Blutcholesterinspiegels beträgt bis zu 30 g pro Tag. Die dabei zu verzeichnenden Nebenwirkungen sind minimal, und es kann deshalb mit Sicherheit davon ausgegangen werden, daß es in Einnahmemengen von bis zu einem Gramm pro Tag als nebenwirkungsfrei anzusehen ist. In dieser Dosierung ist der Einfluß auf einen als normal anzusehenden Blutfettspiegel vernachlässigbar klein.

Die Austauschkapazität von Colestipolhydrochlorid wurde durch Rücktitration mit einer NaOH-Lösung nach Umsetzung mit einer Salzsäurelösung genau bekannter Konzentration zu 8,1 Milliäquivalenten bestimmt. Dies ist im Vergleich zu anderen mehr technischen Ionenaustauschern ein recht hoher Wert und entspricht z.B. 1,6 g Ibuprofen, einem häufig verwendeten Schmerzmittel, das zu 200 mg pro Einzeldosis rezeptfrei im Handel ist.
Der Komplex Colestipol/Ibuprofen entsprechend dieser Einzeldosis hat damit bei maximaler Beladung ein Gesamtgewicht von nur 325 mg, und es ist deshalb kein Problem, diese Menge in eine handelsübliche Kapsel (z.B. Hartgelatinekapsel) abzufüllen.

Colestipolhydrochlorid gehört aufgrund seiner tertiären Aminogruppen zu den schwachen Anionenaustauschern.

Bei Beladung mit Säuren werden
a. die basischen Stickstoffatome durch Protonierung in das entsprechende Ammoniumsalz überführt
   und
b. die Chlorionen der als Hydrochlorid vorliegenden Stickstoffatome gegen das neue Säureanion ausgetauscht.

Da der größere Teil der Stickstoffatome im Colestipolhydrochlorid im unprotonierten Zustand vorliegt, muß Colestipol zur Beladung mit der freien Wirkstoffsäure umgesetzt werden. Üblicherweise werden Ionenaustauscher beladen, indem man sie in Kontakt mit einer Lösung der Substanzen bringt, mit denen der jeweilige Austauscher beladen werden soll. Da die unprotonierten Stickstoffatome schwache Basen sind und die Wirkstoffe im allgemeinen nur schwache Säuren, liegt das Gleichgewicht dieser Reaktion aufgrund chemisch-physikalischer Gesetzmäßigkeiten auf der Seite der Reaktanden, so daß in diesem speziellen Fall Colestipol nur zu einem kleinen Bruchteil seiner maximalen Austauschkapazität mit Wirkstoff beladen werden kann.

Es wurde nun ebenfalls überraschenderweise gefunden, daß flüssige bzw. unzersetzt schmelzende Wirkstoffsäuren ohne Verwendung von Lösemitteln innerhalb von ca. 15 Minuten aus der flüssigen Phase an Colestipol gebunden werden können. Unter diesen Bedingungen ist die Beladung bis zur theoretischen Beladungsgrenze ohne Probleme möglich.
Dieses Verfahren ist mit allen Wirkstoffen möglich, die unzersetzt unterhalb der Zersetzungstemperatur des Colestipols bei ca. 180 °C selbst unzersetzt schmelzen. Das Reaktionsprodukt ist wiederum, wie das Colestipol selbst, ein frei fließendes Granulat. Nur unter dem Mikroskop ist die Beladung an einer Vergrößerung der einzelnen nahezu sphärischen Granulatteilchen zu sehen.
Bei Wirkstoffen, die wegen ihrer Thermolabilität nicht in ihre Schmelze überführt werden können, bietet sich noch die Möglichkeit an, sie mit zur Schmelzpunktsdepression geeigneten Hilfsstoffen zu mischen. Natürlich darf der dabei möglicherweise nicht absorbierbare Hilfsstoff sich nicht störend auf das Freisetzungsverhalten und andere wichtige Eigenschaften der Arzneiform auswirken.
Während das unbeladene Colestipol mit Kugelmühlen und verwandten Verfahren praktisch nicht vermahlbar ist, gelingt dies leicht mit beladenem Colestipol. Die Ursache ist eine durch die Beladung stark erhöhte innere Spannung, die auch Ursache der Teilchenvergrößerung ist.

Zur Herstellung der fertigen Arzneiform kann das beladene Colestipol einfach unvermahlen in eine der üblichen Hartgelatinekapseln gefüllt werden oder nach Vermahlung zu allen anderen bekannten festen oralen Darreichungsformen - z.B. Tabletten - weiterverarbeitet werden.
Alternativ dazu kann das Colestipol auch in feuchter Form - auch Wasser führt zu einer starken Quellung - vermahlen und erst nach Trocknung mit Wirkstoff beladen werden.

Die Bindung an Colestipol ist eine ausgezeichnete Möglichkeit, geeignete und bei Raumtemperatur flüssige bzw. niedrig schmelzende - und damit aus galenischer Sicht problematische Wirkstoffe - in eine feste, temperaturunempfindliche und leicht weiterzuverarbeitende Form zu überführen.

Saure Arzneistoffe haben im ebenfalls sauren Magensaft eine schlechte Löslichkeit. Dies ist von erheblichem Nachteil, wenn ein schneller Wirkungseintritt erwünscht ist. Es zeigte sich nun in entsprechenden Versuchen, daß der Wirkstoff im Magensaft eine übersättigte Lösung bildet und unter Umständen sogar Wirkstoffe, die bei der Körpertemperatur von 37 °C kristallin vorliegen, in flüssiger Form freigesetzt werden.

Wegen der erhöhten thermodynamischen Aktivität von unterhalb ihres Schmelzpunktes flüssig vorliegenden Wirkstoffen, führt auch dies zu einem erhöhten Anteil an gelöstem Wirkstoff und ermöglicht damit eine beschleunigte Resorption und einen damit verbundenen früheren Wirkungseintritt.

Dies sei am Beispiel des S(+)-Ibuprofen demonstriert. Während das racemische Ibuprofen einen Schmelzpunkt von 77 - 78 °C aufweist, schmilzt das alleinig wirksame S(+)--Enantiomere schon bei 52 - 53 °C. Wegen dieses niedrigen Schmelzpunktes, und einiger weiterer ungünstiger physikalischer Eigenschaften ist es eine für die herkömmliche Tablettierung - was die Handhabung angeht - sehr problematische Substanz. Die resultierenden Tabletten haben - wie alle anderen denkbaren, die freie Wirkstoffsäure enthaltenden, festen Arzneiformen den Nachteil, daß selbst eine nur kurzfristige Einwirkung von Temperaturen oberhalb des Schmelzpunktes von S(+)-Ibuprofen die Arzneiform bezüglich ihrer physikalischen Eigenschaften und speziell der Wirkstofffreisetzung irreversibel schädigt.

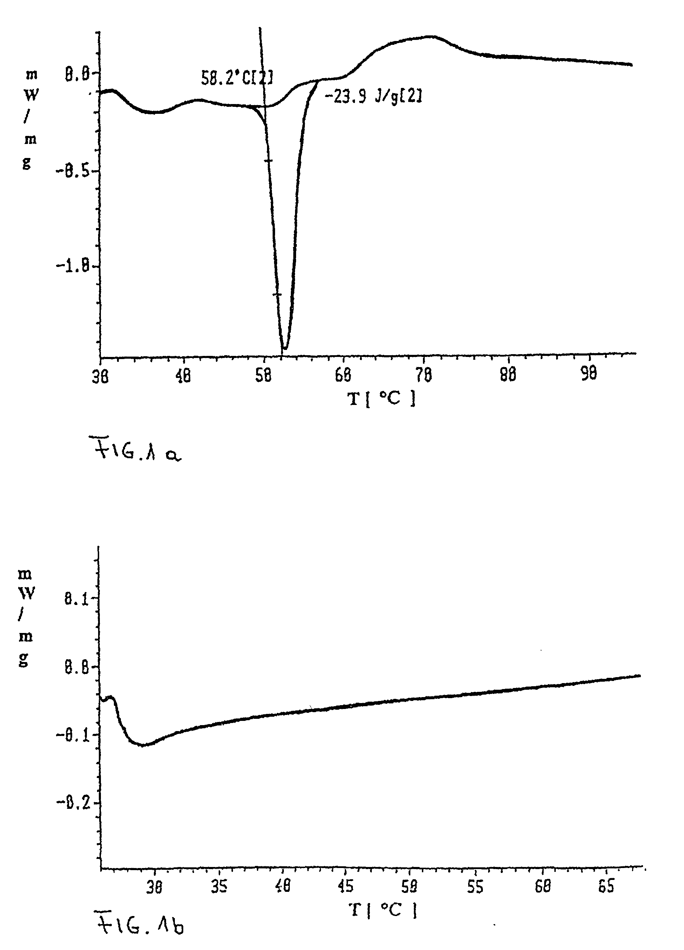

Nach der Bindung an das Colestipol kann mit entsprechenden DSC-(Differential scanning calorimetry) Untersuchungen kein schmelzbares Ibuprofen mehr nachgewiesen werden (Figur 1a und 1b). Selbst nach mehreren Jahren Lagerung kann mit diesem Analysenverfahren kein freies und damit schmelzbares Ibuprofen nachgewiesen werden. Dies zeigt, daß der Komplex über eine für die Verwendung als Arzneimittel ausreichende Stabilität verfügt.

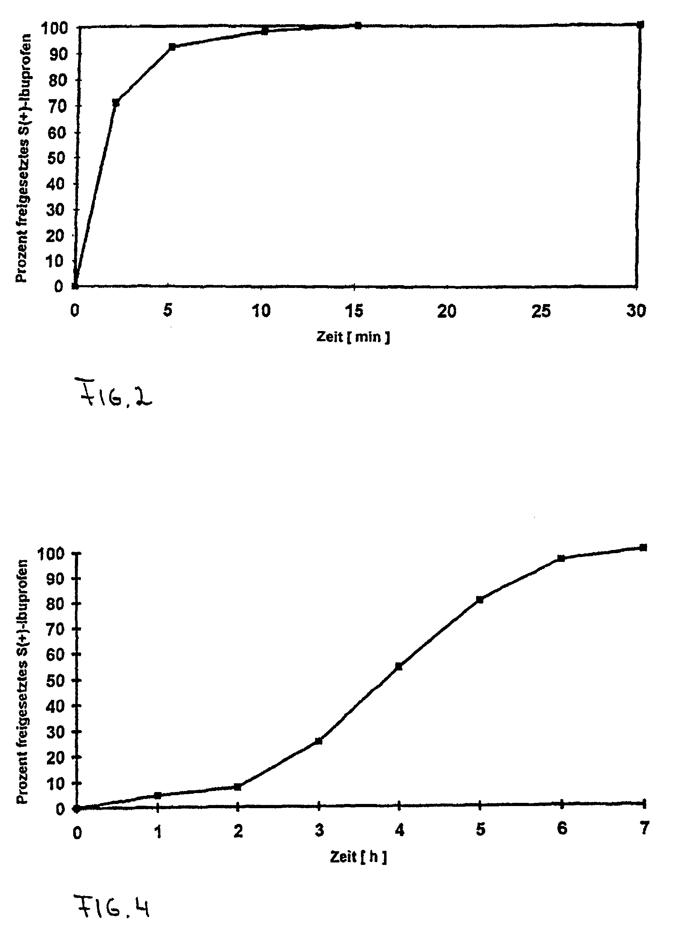

Fig. 2 zeigt die Freisetzung von an Colestipol gebundenen 200 mg S(+)-Ibuprofen in 900 ml eines Phosphatpuffers mit einem pH-Wert von 6,8.
Der Versuch wurde in einem Dissolution Tester nach der Paddle Methode bei einer Temperatur von 37°C durchgeführt und zeigte, daß schon nach 5 Minuten über 80% des in der Kapsel enthaltenen Wirkstoffs freigesetzt werden.

Damit sind betreffend der Freisetzungsgeschwindigkeit alle Anforderungen an eine schnell freisetzende Arzneiform erfüllt.

Um die Sättigungslöslichkeit von freiem S(+)-Ibuprofen und an Colestipol gebundenen S(+)-Ibuprofen zu bestimmen, wurden beide Formen in einem genügendem Überschuß zu 100 ml künstlichem Magensaft gegeben und nach der Einstellung des Gleichgewichts die Konzentration an gelöstem S(+)-Ibuprofen gemessen. Es zeigte sich, daß sich beim freien S(+)-Ibuprofen 4,5 ml/100 ml lösten und bei Verwendung des Colestipolkomplexes 13,8 g/100 ml.
Dies bedeutet eine Löslichkeitserhöhung um den Faktor 3.

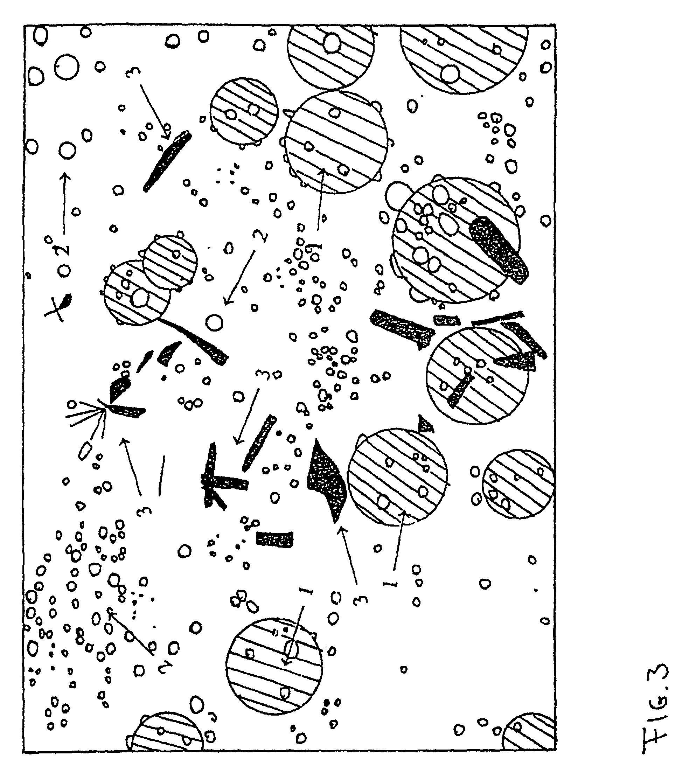

Da bei Verwendung von S(+)-Ibuprofen Pulver davon ausgegangen werden kann, daß die Sättigungslöslichkeit erreicht wird, muß im Falle des Ibuprofen/Colestipol-Komplexes damit die normale Sättigungslöslichkeit überschritten werden. Wenn man die Freisetzung unter dem Mikroskop verfolgt (Figur 3; 1 Colestipolteilchen, 2 flüssiges S(+)-Ibuprofen, 3 rekristallisiertes S(+)-Ibuprofen; Vergrößerung 200 x), erkennt man, daß der Wirkstoff in flüssiger Form freigesetzt wird und nur sporadisch spontan aus dieser flüssigen Phase rekristallisiert. Da die flüssige Phase des bei 37 °C eigentlich festen S(+)-Ibuprofens einen Zustand erhöhter thermodynamischer Aktivität darstellt, erklärt dies zwanglos die höhere Löslichkeit in künstlichem Magensaft.

Diese erhöhte Löslichkeit in Magensaft und die kinetisch nicht gehemmte Freisetzung des Ibuprofens aus dem Komplex ermöglichen eine im Vergleich zu konventionellen Ibuprofenzubereitungen schnellere Wirkstofffreisetzung schon im Magen. Dies ist für ein Schmerzmittel, das zum überwiegenden Teil bei Akutschmerzen wie Kopf- und Zahnschmerzen eingenommen wird, ein nicht zu unterschätzender Vorteil.

Wenn eine schnelle Wirkstofffreisetzung gerade nicht erwünscht ist, lassen sich mit den Colestipol/Wirkstoffkomplexen auch Retardpräparate herstellen. Es bleibt dabei bei niedrig schmelzenden Wirkstoffen immer noch der Vorteil der Temperaturunempfindlichkeit.
Für die Herstellung von solchen Retardpräparaten können prinzipiell alle gängigen, dem Fachmann bekannten, Retardierungsverfahren angewendet werden. Es ist dabei unter Umständen von Vorteil wenn der Komplex in vermahlenem Zustand eingesetzt wird. Dies gilt insbesondere, wenn unter Verwendung von geeigneten und dem Fachmann geläufigen Hilfsstoffen Komprimate hergestellt werden sollen.

Bei S(+)-Ibuprofen hat sich die Granulation des unvermahlenen beladenen Colestipols mit Na-Alginat und anschließender Abfüllung in magensaftlösliche Kapseln als besonders vorteilhaft herausgestellt. Der sich bei Kontakt mit Magensaft bildende Gelfilm des Granulats bildet eine Diffusionsbarriere und bewirkt dadurch die langsame Freisetzung des Ibuprofens über einen Zeitraum bis zu 8 Stunden.
In Figur 4 wird das Ergebnis eines Freisetzungsexperiments nach der sogenannten Half-Change Methode gezeigt. Nach dieser Methode wird die Passage einer Arzneiform durch den Magendarmtrakt durch die eine stufenförmige Erhöhung des pH-Wertes simuliert. Dies wird dadurch erreicht, daß das zu Anfang aus künstlichem Magensaft bestehende Freisetzungsmedium zu den Probenahmezeiten jeweils zur Hälfte gegen künstlichen Darmsaft ausgetauscht wird.
Der Kurvenverlauf zeigt, daß das gesamte in der Arzneiform enthaltene Ibuprofen kontinuierlich über eine Zeit von etwa 8 Stunden freigesetzt wird.

Die am Beispiel S(+)-Ibuprofen gezeigten Ergebnisse lassen sich auch auf andere Wirkstoffe übertragen. Dies gilt insbesonders für Wirkstoffe aus der Gruppe der nichtsteroidalen Antirheumatika wie z.B. Indomethacin, Acemetacin, Sulindac, Tolmetin, Diclofenac, Lonazolac, Ketoprofren, Ibuprofen rac., Flurbiprofen, Fenoprofen, Naproxen, Pirprofen, Indoprofen, Caprofen und Tiaprofensäure, aber auch für Substanzen wie Valproinsäure, ein Antiepileptikum, und Captopril, ein Antihypertensivum.

Die Figuren zeigen zusammenfassend:
Figur 1a
   DSC-Untersuchung an S(+)-Ibuprofen
   Heating rate 10 K/min
   Gasatmosphäre: Luft
Figur 1b
   DSC-Untersuchung am S(+)-Ibuprofen/Colestipol-Komplex Heating rate 10 K/min
   Gasatmosphäre: Luft
Figur 2
   Freisetzungsgeschwindigkeit von an Colestipol gebundenem S(+)-Ibuprofen aus magensaftlöslichen Hartgelatinekapseln
Figur 3
   S(+)-Ibuprofen-Komplex bei Kontakt mit künstlichem Magensaft; mikroskopische Aufnahme, Vergrößerung 200 x.
Figur 4
   In-vitro Freisetzung von S(+)-Ibuprofen nach der
   Half-Change-Methode (n = 6, senkrechte Balken stellen die Standardabweichung dar)

### Beispiele

### 1. Schnell freisetzende S(+)-Ibuprofen-Formulierung

72 g S(+)-Ibuprofen werden geschmolzen und auf 80 °C erwärmt.
Unter Rühren werden 48 g Colestipolhydrochlorid zugegeben und unter weiterem Rühren bzw. Mischen wird diese Mischung 25 Minuten bei 80 °C gehalten. Danach hat sich ein "trokkenes" Granulat gebildet, das nach Abkühlung auf Raumtemperatur zu je 333 mg (200 mg Wirkstoff) in magensaftlösliche Gelatinekapseln dosiert wird.

### 2. Langsam freisetzende S(+)-Ibuprofen-Formulierung

Zu 110 g des mit S(+)-Ibuprofen beladenen Granulate aus Beispiel 1 werden 27,5 g Na-Alginat gegeben und sorgfältig gemischt. Danach werden 138 g entionisiertes Wasser zugegeben und die sich bildende teigartige Masse durch Kneten homogenisiert. Aus der teigigen Masse wird ein Stranggranulat (Durchmesser 0,7 mm, Länge 1 mm) hergestellt und das getrocknete Granulat in Dosen zu je 432 mg (entspr. 200 mg Wirkstoff) in magensaftlösliche Gelatinekapseln abgefüllt.

### 3. Schnell freisetzende Captopril-Formulierung

64 g Captopril werden geschmolzen und auf 100 - 110 °C erwärmt.

Unter Rühren werden 48 g Colestipolhydrochlorid zugegeben. Unter weiterem Rühren bzw. Mischen wird diese Mischung 25 Minuten bei 100 - 110 °C gehalten. Danach hat sich ein "trockenes" Granulat gebildet, das nach Abkühlung auf Raumtemperatur zu je 43 mg (25 mg Wirkstoff) in magensaftlösliche Gelatinekapseln dosiert wird.

## Patentansprüche

1. Orale Arzneiform mit einem Träger für sauren Wirkstoff, bei dem der Wirkstoff über mindestens eine freie Carboxylgruppe verfügt, die ionisch an ein Polymer mit tertiären Aminogruppen gebunden ist, **dadurch gekennzeichnet, daß** das Polymer durch Colestipol bzw. Colestipolhydrochlorid in einer unterhalb seiner eigenen pharmazeutischen Wirksamkeit liegenden Menge gebildet wird, an das der Wirkstoff ohne Lösungsmittelzusatz unter Bildung eines rieselfähigen Granulats angelagert worden ist.

2. Orale Arzneiform nach Anspruch 1, **dadurch gekennzeichnet, daß** der Wirkstoff zu der Gruppe der nichtsteroidalen Antirheumatika gehört.

3. Orale Arzneiform nach Anspruch 2, **dadurch gekennzeichnet, daß** der Wirkstoff entweder zu den Anthranilsäure-, den Essigsäure- oder den Propionsäurederivaten gehört.

4. Orale Arzneiform nach Anspruch 1, **dadurch gekennzeichnet, daß** der Wirkstoff ein Racemat darstellt.

5. Orale Arzneiform nach Anspruch 1, **dadurch gekennzeichnet, daß** der Wirkstoff über mindestens ein chirales Zentrum verfügt und im wesentlichen enantiomerenrein vorliegt.

6. Orale Arzneiform nach Anspruch 3, **dadurch gekennzeichnet, daß** der Wirkstoff Ibuprofen ist.

7. Orale Arzneiform nach Anspruch 6, **dadurch gekennzeichnet, daß** der Wirkstoff S(+)-Ibuprofen ist.

8. Orale Arzneiform nach Anspruch 1, **dadurch gekennzeichnet, daß** der Wirkstoff Valproinsäure ist.

## Claims

1. Oral administration form having a carrier for acid active substance wherein the active substance has at least one free carboxyl group ionically bound to a polymer having tertiary amino groups, **characterized in that** the polymer is formed by colestipol or colestipol hydrochloride in an amount lying below its own pharmaceutical efficacy, to which the active substance has been attached without the addition of solvents, thus forming a free-flowing granulate.

2. The oral administration form according to claim 1 **characterized in that** the active substance belongs to the group of the nonsteroidal antirheumatics.

3. The oral administration form according to claim 2 **characterized in that** the active substance either belongs to the derivatives of anthranilic acid, acetic acid, or propionic acid.

4. The oral administration form according to claim 1 **characterized in that** the active substance represents a racemate.

5. The oral administration form according to claim 1 **characterized in that** the active substance has at least one chiral center and is present in a substantially pure form with regard to enantiomers.

6. The oral administration form according to claim 3 **characterized in that** the active substance is ibuprofen.

7. The oral administration form according to claim 6 **characterized in that** the active substance is S (+)-ibuprofen.

8. The oral administration form according to claim 1 **characterized in that** the active substance is valproic acid.

9. The oral administration form according to claim 1 **characterized in that** the active substance is captopril.

10. The oral administration form according to claim 1 **characterized in that** it is a chewable tablet or a chewing gum.

11. The oral administration form according to claim 1 **characterized in that** it is a capsule or a tablet.

12. A process for the production of an oral administration form according to one or several of claims 1-11, **characterized in that** the active substance is bound from a liquid solvent-free phase to the basic groups of colestipol or colestipol hydrochloride.

13. A process for the production of an oral administration form according to one or several of claims 1-11, **characterized in that** the active substance is mixed with an auxiliary agent for melting-point depression, and that it is bound to the basic groups of colestipol or colestipol hydrochloride from said mixture.

## Revendications

1. Forme galénique à administrer par voie orale, ayant un véhicule pour un principe actif acide, dans laquelle le principe actif dispose d'au moins un groupe carboxylique libre, qui est lié de façon ionique à un polymère avec des groupes amine tertiaire, **caractérisée en ce que** le polymère est formé par du colestipol ou un chlorhydrate de colestipol en une quantité inférieure à sa quantité efficace propre sur le plan pharmaceutique, polymère sur lequel le principe actif a été fixé par addition sans ajouter de solvant et avec formation d'un granulat capable de s'écouler.

2. Forme galénique à administrer par voie orale selon la revendication 1, **caractérisée en ce que** le principe actif appartient au groupe des anti-rhumatismaux non stéroïdiens.

3. Forme galénique à administrer par voie orale selon la revendication 2, **caractérisée en ce que** le principe actif appartient aux dérivés de l'acide anthranilique, de l'acide acétique ou de l'acide propionique.

4. Forme galénique à administrer par voie orale selon la revendication 1, **caractérisée en ce que** le principe actif représente un racémate.

5. Forme galénique à administrer par voie orale selon la revendication 1, **caractérisée en ce que** le principe actif dispose d'au moins un centre chiral et existe essentiellement en tant qu'énantiomère pur.

6. Forme galénique à administrer par voie orale selon la revendication 3, **caractérisée en ce que** le principe actif est l'ibuprofène.

7. Forme galénique à administrer par voie orale selon la revendication 6, **caractérisée en ce que** le principe actif est le S (+)-ibuprofène.

8. Forme galénique à administrer par voie orale selon la revendication 1, **caractérisée en ce que**, le principe actif est l'acide valproïque

9. Forme galénique à administrer par voie orale selon la revendication 1, **caractérisée en ce que** le principe actif est le captopril.

10. Forme galénique à administrer par voie orale selon la revendication 1, **caractérisée en ce qu'**elle constitue un comprimé à mâcher ou une gomme à mâcher.

11. Forme galénique à administrer par voie orale selon la revendication 1, **caractérisée en ce qu'**elle constitue une capsule ou un comprimé.

12. Procédé de préparation d'une forme galénique à administrer par voie orale, selon l'une ou plusieurs des revendications 1 à 11, **caractérisé en ce que** le principe actif issu d'une phase liquide, exempte de solvent est lié aux groupes basiques du colestipol ou du chlorhydrate de colestipol.

13. Procédé de préparation d'une forme galénique à administrer par voie orale, selon l'une ou plusieurs des revendications 1 à 11, **caractérisé en ce que** le principe actif est mélangé avec un adjuvant afin d'abaisser le point de fusion et à partir de ce mélange, il est lié aux groupes basiques du colestipol ou du chlorhydrate de colestipol.
